# EUROPEAN PATENT APPLICATION

(11) **EP 2 409 685 A2**
(43) Date of publication of application: **25.01.2012**
(21) Application number: 11174395.1
(22) Date of filing: 18.07.2011
(51) Int. Cl.: A61K 9/00, A61K 9/20, A61K 31/4365, A61P 7/02, A61P 9/00, A61K 9/50

(54) **Orally-disintegrating formulations of prasugrel**

(30) Priority: 19.07.2010 TR 201005900; 17.08.2010 TR 201006802
(71) Applicant: Sanovel Ilac Sanayi ve Ticaret A.S., 34398 Istanbul (TR)
(72) Inventor: Cifter, Ümit, 34398 Istanbul (TR); Türkyilmaz, Ali, 34398 Istanbul (TR); Öner, Levent, 34398 Istanbul (TR)
(74) Representative: Sevinç, Erkan

(57) **Abstract**

The present invention relates to an orally-disintegrating pharmaceutical formulation, comprising prasugrel or a pharmaceutically acceptable derivative thereof and one or more pharmaceutically acceptable excipient(s).

## Description

### Field of Invention

The present invention relates to a formulation of prasugrel or a pharmaceutically acceptable salt thereof. The present invention particularly relates to an orally-disintegrating tablet formulation of prasugrel, which is stable against ambient influences.

### Background of Invention

Prasugrel is a member of the thienopyridine class and inhibits the activation and aggregation of platelets by means of P2Y12 and ADP receptors. Its chemical designation is 2-acetoxy-5-(α-cyclopropylcarbonyl-2-fluorobenzyl)-4,5,6,7-tetrahydrothieno[3,2-c]pyridine, with the chemical structure illustrated below with Formula 1.

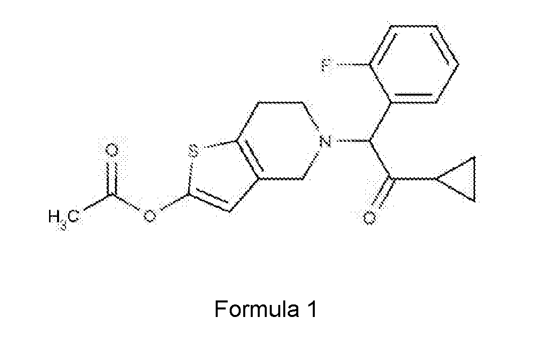

The prasugrel hydrochloride salt is marketed under the trademark Effient^{®} in 5 or 10 mg unit dosage forms. It is initially administered in a dosage amount of 60 mg, as a loading dose. It is used in preventing or treating thrombosis and cardiovascular diseases.

The prasugrel hydrochloride salt is a white solid. Prasugrel hydrochloride is well soluble at pH 2, weakly soluble at pH 3 to 4, and is substantially insoluble at pH 6 to 7.5.

Searching the patent literature reveals various patents in relation to prasugrel.

Patent application EP1298132, for instance, discloses a prasugrel hydrochloride salt, a process for obtaining this salt, and its use for thrombosis and embolism.

Patent application EP1728794 discloses a prasugrel maleat salt, a process for obtaining this salt, and its use for thrombosis and embolism.

Patent application WO2006135605 discloses a formulation containing prasugrel hydrochloride, packaged in an air and moisture impervious gas-inerted blister pack.

Patent application WO2006138317 discloses a dosage administration required for the loading dose and following doses of prasugrel.

Patent application WO2008073759 discloses a tablet or capsule formulation containing prasugrel, packaged in an air- and humidity-impermeable blister under a gas with positive fluidity pressure in order to reduce the amount of oxygen and humidity generated during packaging.

Many tablet formulations of prasugrel are disclosed in aforesaid patents. This active pharmaceutical agent used for treating many diseases, however, is preferred in an orally-disintegrating form. Such preparations are advantageous for use in younger, elder, and noncompliant patients. Orally-disintegrating dosage forms are convenient when drinking water is not available or preferable.

Developing orally-disintegrating formulations is difficult due to several different reasons. First of all, the time required for the disintegration of the dosage form in the oral cavity under the presence of saliva is shorter than the time required by the stomach. Therefore, these formulations are quite porous and therefore are not too hard. Such porous formulations are prone to be susceptible against humidity. As a result of this, they may lead to some stability problems. In addition, measures must be taken while preparing, packaging, handling and storing finished dosage forms of orally-disintegrating formulations.

For this reason, orally-disintegrating compositions of prasugrel or a pharmaceutically acceptable salt thereof is desired, which would overcome the drawbacks referred to hereinabove and would let the active agent be disintegrated and dissolved in the oral cavity.

In addition to the stability problems which may arise from the porous structure of this dosage form, other stability problems are frequently encountered in prasugrel formulations under the influence of ambiance and physical conditions. Prasugrel is an active agent that is highly-susceptible to air and humidity conditions. When prasugrel is initially exposed to air and humidity, it degrades structurally and develops behavioral changes. As a result of this fact, two main problems emerge. The first problem is that the stability of prasugrel products developed is not of desired level and the shelf life thereof is shortened. The second problem is that prasugrel is reactive against the excipients employed in developing formulations containing prasugrel. This fact causes impurities and unwanted components to be included into the formulation.

In result, due to the abovementioned drawbacks, a novelty is required in the art of prasugrel formulations, used for preventing and treating thrombosis and cardiovascular diseases.

### Object and Brief Description of Invention

The present invention provides a prasugrel formulation, eliminating all aforesaid problems and bringing additional advantages to the relevant prior art.

Accordingly, the main object of the present invention is to obtain an orally-disintegrating novel formulation with antithrombotic activity.

Another object of the present invention is to obtain a stable prasugrel formulation with high bioavailability.

A further object of the present invention is to provide a high dissolution rate for such stable prasugrel formulations with high bioavailability.

Another object of the present invention is to avoid any aggregation of ingredients of the composition and provide a desired level of flowability during production, thanks to convenient excipients used while the formulation is obtained.

An orally-disintegrating pharmaceutical formulation has been developed to carry out any objects, referred to above and to emerge from the following detailed description.

In a preferred embodiment according to the present invention, said novelty is realized with prasugrel or a pharmaceutically acceptable derivative thereof, together with one or more pharmaceutically acceptable excipient(s). The formulation disintegrates in the oral cavity in 90 seconds, preferably in 40 seconds.

In a preferred embodiment according to the present invention, prasugrel granules with at least one pullulan-containing coating layer are provided in said formulation.

In another preferred embodiment according to the present invention, the amount of pullulan in said coating layer with respect to the total granule amount is 0.01 to 20% by weight, preferably 0.1 to 10% by weight, and more preferably 0.2 to 4% by weight.

In another preferred embodiment according to the present invention, said prasugrel salt is a hydrochloride salt or maleat salt.

In another preferred embodiment according to the present invention, prasugrel or a pharmaceutically acceptable salt thereof is included in the formulation at 1 to 80% by the total weight of formulation, preferably at 10 to 60% by the total weight of formulation, and more preferably 20 to 50% by the total weight of formulation.

In another preferred embodiment according to the present invention, one or more pharmaceutically acceptable excipient(s) are selected from the group consisting of super disintegrants, diluents, binders, lubricants, glidants, sweeteners, flavoring agents, preserving agents and coloring agents.

In another preferred embodiment according to the present invention, said super disintegrants are selected from the group consisting of at least one or a mixture of croscarmellose sodium, crospovidone, low-substituted hydroxypropyl cellulose, sodium starch glycolate, and soy polysaccharides (Emcosoy^{®}).

In another preferred embodiment according to the present invention, the weight proportion of prasugrel to super disintegrants is between 1:10 and 10:1.

In another preferred embodiment according to the present invention, said super disintegrant is preferably croscarmellose sodium.

In another preferred embodiment according to the present invention, said diluent is preferably mannitol and/or microcrystalline cellulose.

In another preferred embodiment according to the present invention, said binder is preferably polyvinylpyrrolidone and/or hydroxypropyl methyl cellulose.

In another preferred embodiment according to the present invention, said glidant and lubricant are preferably magnesium stearate and sodium lauryl sulfate.

In another preferred embodiment according to the present invention, said glidant is preferably colloidal silicone dioxide.

In another preferred embodiment according to the present invention, said sweetener is preferably aspartam, sucralose and/or saccharine.

In another preferred embodiment according to the present invention, said flavoring agents are preferably menthol and/or fruit extracts.

In another preferred embodiment according to the present invention, said pharmaceutical formulation is consisted of the following ingredients only:
(a) prasugrel or a pharmaceutically acceptable salt thereof at approximately 2 to 60% by weight;
(b) croscarmellose sodium at approximately 0.1 to 30% by weight,
(c) mannitol at approximately 1 to 60% by weight,
(d) microcrystalline cellulose at approximately 1 to 30% by weight,
(e) polyvinylpyrrolidone at approximately 1 to 20% by weight,
(f) colloidal silicone dioxide at approximately 0.01 to 5% by weight,
(g) magnesium stearate and sodium lauryl sulfate at approximately 0.1 to 5% by weight,
(h) saccharine sodium at approximately 0.01 to 5% by weight,
(i) menthol and/or fruit extracts at approximately 0.01 to 5% by weight.

In a further preferred embodiment according to the present invention, said formulation is in the form of a tablet.

Another preferred embodiment according to the present invention provides a method for preparing said pharmaceutical formulation, this method comprising the steps of
(a) admixing prasugrel or a pharmaceutically acceptable salt of prasugrel with other excipients;
(b) blending this mixture with a lubricant and a glidant; and
(c) compressing the blended mixture in order to obtain tablets.

### Detailed Description of Invention

The present invention is described in more details with the following example. This example is not limiting the scope of the present invention and must be considered under the light of the foregoing detailed disclosure. It is obvious that those skilled in the relevant art can produce similar embodiments under the light of the foregoing disclosures by making many adaptations on both the materials and methods, without departing from the scope of the present invention.

This orally-disintegrating tablet composition composed of the followings is designed to minimize the disintegration time of tablets and maximize the mechanical resistance.

### Example

An orally-disintegrating tablet composition is composed of the followings:
(a) prasugrel or a pharmaceutically acceptable salt of prasugrel at approximately 2 to 60% by weight;
(b) croscarmellose sodium at approximately 0.1 to 30% by weight,
(c) mannitol at approximately 1 to 60% by weight,
(d) microcrystalline cellulose at approximately 1 to 30% by weight,
(e) polyvinylpyrrolidone at approximately 1 to 20% by weight,
(f) colloidal silicone dioxide at approximately 0.01 to 5% by weight,
(g) magnesium stearate and sodium lauryl sulfate at approximately 0.1 to 5% by weight,
(h) saccharine sodium at approximately 0.01 to 5% by weight,
(i) menthol and/or fruit extracts at approximately 0.01 to 5% by weight.

The production process is conducted preferably by admixing the ingredients, blending this admixture with lubricant(s) and glidant(s), and compressing the blended mixture into tablets.

The orally-disintegrating formulations according to the present invention can be prepared by techniques such as direct compression, dry granulation, wet granulation, etc. well known to those skilled in the art. Orally-disintegrating compositions according to the present invention can further be prepared by means of technologies such as spray drying, freeze drying, floss formation process, molding, Zydis^{®} technology, Flashtab technology, OraSolv^{®} technology, DuraSolv^{®} technology, Wowtab^{™} (nonaqueous immediately-soluble tablet) technology and other similar technologies. Prasugrel or a pharmaceutically acceptable salt thereof and other excipients are preferably mixed together and then the resulting mixture is compressed to give tablets. Said tablets are packaged into alu-alu blister.

Coated prasugrel active agent is used in the formulation. Accordingly, a coating process is conducted that efficiently prevents the contact of such granules with air and humidity in order to provide stability for the granules of prasugrel or a pharmaceutically acceptable salt of prasugrel. The process steps are as follows. Pullulan or an additional coating material is dissolved in a proper solvent. The solution obtained is spray-coated onto the granules comprising prasugrel or a pharmaceutically acceptable salt of prasugrel, and thus the coating operation is carried out. Then the coated granules are dried. This coating layer may optionally be a single layer or a multilayer. Coated prasugrel granules can be used in the content of medicaments which are efficient in preventing or treating thrombosis and cardiovascular diseases. Orally-disintegrating tablets can be compressed by making use of coated prasugrel granules and convenient excipients.

### Disintegration Test Results

| Samples | Disintegration time |
|---|---|
| Sample 1 | 0:00:19 |
| Sample 2 | 0:00:22 |
| Sample 3 | 0:00:24 |
| Sample 4 | 0:00:17 |
| Sample 5 | 0:00:21 |
| Sample 6 | 0:00:16 |

Disintegration methods is performed according to EP pharmacopoeias.

The orally-disintegrating pharmaceutical formulation obtained has surprisingly shown fairly-good dissolution rates, stability, and oral bioavailability. Coated particles or granules bring the stability of the formulation to a desired level. This operation also avoids the aggregation of particles or granules and any reduction in their flowability during production.

The present invention is used for treating thrombosis and cardiovascular diseases.

Orally-disintegrating formulations according to the present invention comprises tablets, single-dose packages, mini tablets, and multilayered and multicoated tablets, pellets, or powders formulated according to the standard methods of the relevant art. The formulation is preferably in the form of tablets.

The orally-disintegrating tablet composition according to the present invention is preferably in the form of a disk, circle, round, sphere, donut, bar, polygon, ellipse etc.

The term granule, as used herein, means a powder, particle, or pellet form of prasugrel or a pharmaceutically acceptable salt of prasugrel.

The pharmaceutical formulations according to the present invention may also comprise one or more pharmaceutically acceptable excipient(s). Such pharmaceutically acceptable convenient excipients include, but are not restricted to fillers, glidants, lubricants, disintegrants, surface active agents etc. and the mixtures thereof.

Convenient binders include, but are not restricted to, polyvinylpyrrolidone, polyethylene glycol, polyvinyl alcohol, hydroxypropyl methyl cellulose, carboxymethyl cellulose, methyl cellulose and other cellulose derivatives, gelatin, polyvinyl alcohol, carrageenan, guar gum, xanthan gum, and the mixtures thereof.

Suitable lubricants include, but are not limited to, magnesium stearat, calcium stearat, sodium stearyl fumarate, sodium lauryl sulfate, stearic acid, etc. and the mixtures thereof.

Whilst preferred lubricants are magnesium stearat, other less hydrophobic lubricants may be employed in certain circumstances to adversely effect the hydrophobicity, e.g. a combination of magnesium stearate and sodium lauryl sulfate.

Suitable lubricants include, but are not restricted to, colloidal silicone dioxide, talk, aluminum silicate, etc., and the mixtures thereof.

The orally-disintegrating compositions according to the present invention can also include sweeteners and flavoring agents to enhance patient compliance.

Convenient sweeteners include, but are not restricted to, aspartam, sucralose, saccharine; glucose, lactose, fructose and other sugars, and mannitol, sorbitol, xylitol, erythritol, and other sugar alcohols, etc., and the mixtures thereof.

Convenient flavoring agents include, but are not restricted to, menthol, peppermint, cinnamon, chocolate, vanilin, and fruit extracts such as cherry, orange, strawberry, grape, and the mixtures thereof.

Suitable preservatives include, but are not restricted to, methyl paraben and propyl paraben and salts thereof (e.g. sodium or potassium salts), sodium benzoate, citric acid, benzoic acid, butylated hydroxytoluene and butylated hydroxyanisole, and the mixtures thereof.

Suitable coloring agents include, but are not restricted to, iron oxide, FD&C (Food, Drug and Cosmetic) dyes, ponceau, etc. and the mixtures thereof.

## Claims

1. An orally-disintegrating pharmaceutical formulation, comprising prasugrel or a pharmaceutically acceptable derivative thereof and one or more pharmaceutically acceptable excipient(s) wherein the formulation disintegrates within up to 90 seconds, preferably 40 seconds in oral cavity.

2. The pharmaceutical formulation according to any of the preceding claims, comprising prasugrel granules having at least one coating layer containing pullulan.

3. The pharmaceutical formulation according to any of the preceding claims, wherein the amount of pullulan in said coating layer with respect to the total prasugrel granule amount is 0.01 to 20% by weight, preferably 0.1 to 10% by weight, and more preferably 0.2 to 4% by weight.

4. The pharmaceutical formulation according to any of the preceding claims, wherein said prasugrel salt is a hydrochloride salt or a maleat salt.

5. The pharmaceutical formulation according to any of the preceding claims, wherein prasugrel or a pharmaceutically acceptable salt thereof is included in the formulation at 1 to 80% by the total weight of formulation, preferably at 10 to 60% by the total weight of formulation, and more preferably 20 to 50% by the total weight of formulation.

6. The pharmaceutical formulation according to any of the preceding claims, wherein one or more pharmaceutically acceptable excipients are selected from the group consisting of super disintegrants, diluents, binders, lubricants, glidants, sweeteners, flavoring agents, preserving agents and coloring agents.

7. The pharmaceutical formulation according to any of the preceding claims, wherein super disintegrants are selected from the group consisting of at least one or a mixture of croscarmellose sodium, crospovidone, low-substituted hydroxypropyl cellulose, sodium starch glycolate, and soy polysaccharides.

8. The pharmaceutical formulation according to any of the preceding claims, wherein the weight ratio of prasugrel to super disintegrants is in the range of 1:10 to 10:1.

9. The pharmaceutical formulation according to any of the preceding claims, wherein said super disintegrant is preferably croscarmellose sodium.

10. The pharmaceutical formulation according to any of the preceding claims, wherein said diluent is preferably mannitol and/or microcrystalline cellulose.

11. The pharmaceutical formulation according to any of the preceding claims, wherein said binder is preferably polyvinylpyrrolidone and/or hydroxypropyl methyl cellulose.

12. The pharmaceutical formulation according to any of the preceding claims, wherein said lubricant is preferably magnesium stearate and sodium lauryl sulfate.

13. The pharmaceutical formulation according to any of the preceding claims, wherein said glidant is preferably colloidal silicone dioxide.

14. The pharmaceutical formulation according to any of the preceding claims, wherein said sweetener is preferably aspartam, sucralose and/or saccharine.

15. The pharmaceutical formulation according to any of the preceding claims, wherein said flavoring agent is preferably menthol and/or fruit extracts.

16. The pharmaceutical formulation according to any of the preceding claims, composed of the following ingredients only:
(a) prasugrel or a pharmaceutically acceptable salt of prasugrel at approximately 2 to 60% by weight;
(b) croscarmellose sodium at approximately 0.1 to 30% by weight,
(c) mannitol at approximately 1 to 60% by weight,
(d) microcrystalline cellulose at approximately 1 to 30% by weight,
(e) polyvinylpyrrolidone at approximately 1 to 20% by weight,
(f) colloidal silicone dioxide at approximately 0.01 to 5% by weight,
(g) magnesium stearate and sodium lauryl sulfate at approximately 0.1 to 5% by weight,
(h) saccharine sodium at approximately 0.01 to 5% by weight,
(i) menthol and/or fruit extracts at approximately 0.01 to 5% by weight.

17. A process for preparing the pharmaceutical formulation according to any of the preceding claims, comprising the steps of
(a) admixing prasugrel or a pharmaceutically acceptable salt of prasugrel with other excipients;
(b) blending this mixture with a lubricant and a glidant; and
(c) compressing the blended mixture in order to obtain tablets.

18. The pharmaceutical formulation according to any of the preceding claims in the form of tablets.

19. The pharmaceutical formulation according to any of the preceding claims, wherein said tablets are into the alu-alu blister.

20. The pharmaceutical formulation according to any of the preceding claims for preventing or treating thrombosis and cardiovascular diseases in mammalians and particularly in humans.
